# EUROPEAN PATENT APPLICATION

(11) **EP 4 470 523 A1**
(43) Date of publication of application: **04.12.2024**
(21) Application number: 23382518.1
(22) Date of filing: 31.05.2023
(51) Int. Cl.: A61K 9/00, A61K 9/107, A61K 47/22, A61K 47/24, A61P 17/14, A61K 31/58

(54) **TOPICAL PHARMACEUTICAL COMPOSITIONS**

(71) Applicant: Galenicum Health SLU, 08950 Esplugues de Llobregat (ES)
(72) Inventor: TORREJÓN NIETO, Javier, 08950 Esplugues de Llobregat (ES); Capdevila Bellart, Jordi, 08950 Esplugues de Llobregat (ES); Tampucci, Silvia, 33-56126 Pisa (IT); Monti, Daniela, 33-56126 Pisa (IT); Chetoni, Patrizia, 33-56126 Pisa (IT)
(74) Representative: Torrejón-Nieto, Javier

(57) **Abstract**

The invention relates to a topical pharmaceutical composition for the treatment and/or prevention of androgenetic alopecia comprising: 0.03-0.3% w/w of an active ingredient, wherein the active ingredient is a 5-alpha reductase inhibitor, pharmaceutically acceptable salts or mixtures thereof; and 1-15% w/w of a first surfactant; wherein the first surfactant is forming nanoparticles having a hydrodynamic diameter between 3-700 nm measured by DLS, wherein said nanoparticles comprise the active ingredient; and wherein the composition is free of alcohol.

## Description

The present invention relates to topical pharmaceutical compositions comprising 5-alpha reductase inhibitors, methods for their preparation and the use of the said compositions for the treatment of androgenetic alopecia.

### STATE OF THE ART

Androgenetic alopecia (AGA) is a common hair loss disorder that affects both men and women, and which is caused by a combination of genetic, hormonal, and environmental factors. In the hair follicles, the conversion of testosterone to dihydrotestosterone (DHT) by the enzyme 5α-reductase and the subsequent complexes formed between DHT and androgen receptors have a strong influence on the hair cycle. An exposure of the hair follicle to high DHT concentrations contributes to the shortening of the active growth phase, resulting in miniaturization of the hair follicles and gradual hair loss.

Current treatments of the AGA include oral and topical formulations of active ingredients such as finasteride or dutasteride. However, systemic exposure to these active ingredients leads to the onset of adverse effects including sexual dysfunction, depression and/or gynecomastia. For this reason, in the case of AGA treatment, a direct targeting to the area of the hair follicle is desirable, as the conversion of DHT from testosterone and the subsequent binding with androgen receptors is settled at the base of the hair follicle.

Nanoparticulated delivery systems have been subject of interest in the formulation of topical compositions since they can improve drug-selective tissue distribution in the hair follicle region, minimizing side effects and reducing the effective dose to be administered. Unfortunately, there is no consensus on the optimal particle size for reaching the base of the follicle, as it is highly dependent on the nature of the delivery systems, the active ingredient and/or the specific combination of excipients.

WO2021229578 A1 describes an oil-in-water emulsion comprising one or more lipophilic bioactive agents, one or more oil solvents, one or more emulsifiers and water, wherein said bioactive agents are substantially dissolved in the internal oil phase of said emulsion, and wherein the mean droplet size of said emulsion is in the range of about 200 to about 1000 nm.

WO19012353 A1 describes a composition for topical application for preventing hair loss comprising dutasteride or pharmaceutically acceptable salt thereof, medium chain triglycerides, castor oil and ethanol, wherein dutasteride contained in the topical application is administered at a daily dose of about 0.1 mg to about 0.5mg.

WO2010015556 A1 discloses a composition for use in the treatment and/or prevention of hair conditions and/or diseases containing hydroxypropyl-chitosan, finasteride, at least one volatile solvent selected from ethanol or isopropanol, and water.

The compositions disclosed in the aforementioned documents contain either high concentrations of active agents, alcohols, and/or oil components. The low aqueous solubility of active ingredients such as finasteride or dutasteride is usually addressed by adding solvents/solubility enhancers such as ethyl alcohol, isopropanol and/or propylene glycol, which lead to adverse effects following repeated applications (e.g., scalp dryness, irritation, burning, redness, allergic contact dermatitis). For instance, commercial topical compositions for the treatment of AGA, such as finasteride spray named Alocare^{®}, present some of the aforementioned drawbacks and do not prevent the systemic absorption of active agents. The said disclosed compositions may also comprise oil components which, despite increasing the solubility of the active ingredient in the formulation, are not pleasant or practical for scalp application, eventually reducing patient compliance.

The present invention is directed towards addressing the shortcomings and challenges outlined above. More specifically, the present invention concerns pharmaceutical compositions that facilitate the deposition of active ingredients for the treatment of AGA within the hair follicles while simultaneously ensuring adequate penetration depth, enhancing drug solubility and reducing the effective dose. The pharmaceutical compositions of the invention are also easy to apply on the scalp of the patient and are able to increase patient convenience and compliance due to an appropriate selection of excipients. In addition, the present invention aims to provide safe and stable pharmaceutical compositions that are straightforward to manufacture at industrial scale.

### SUMMARY OF THE INVENTION

The present invention provides pharmaceutical compositions for improving the distribution and release of active ingredients for the treatment of androgenetic alopecia within the hair follicles. The pharmaceutical compositions of the present invention are also able to avoid or reduce the systemic absorption of the active ingredients while enhancing the drug solubility and reducing the effective dose.

The present invention also provides pharmaceutical compositions which increase patient convenience and compliance. This is achieved by a selection of excipients which allows hair follicle targeting, facilitates the application of the composition on the scalp of the patient and improves user experience.

In addition, the present invention aims to provide safe and stable pharmaceutical compositions that are straightforward to manufacture at industrial scale.

Each of the aspects and embodiments of the present invention described herein may be combined with one or more aspects and embodiments of the present invention.

In a first aspect, the present invention relates to a topical pharmaceutical composition for the treatment and/or prevention of androgenetic alopecia comprising:
a) 0.03-0.3% w/w of an active ingredient, preferably 0.06-0.1% w/w, more preferably 0.09 w/w, wherein the active ingredient is a 5-alpha reductase inhibitor, pharmaceutically acceptable salts or mixtures thereof; and
b) 1-15% w/w of a first surfactant, preferably 2-10% w/w, more preferably 3-5% w/w;
wherein the first surfactant is forming nanoparticles having a hydrodynamic diameter between 3-700 nm measured by DLS, wherein said nanoparticles comprise the active ingredient; and wherein the composition is free of alcohol.

A second aspect of the invention concerns a method for the preparation of the topical pharmaceutical composition according to the first aspect comprising the steps of:
a) heating the first surfactant between 40-60°C;
b) adding the active ingredient, wherein the active ingredient is a 5-alpha reductase inhibitor, pharmaceutically acceptable salts or mixtures thereof;
c) adding water between 40-60ºC; and
d) stirring at room temperature.

A third aspect of the invention relates to a method for the preparation of the topical pharmaceutical composition according to the first aspect comprising the steps of:
a) dissolving the first surfactant;
b) adding the active ingredient, wherein the active ingredient is a 5-alpha reductase inhibitor, pharmaceutically acceptable salts or mixtures thereof under stirring; and
c) continue the stirring.

A fourth aspect of the invention relates to a topical pharmaceutical composition according to the first aspect of the invention for use in the treatment of androgenetic alopecia.

A fifth aspect of the invention relates to a 5-alpha reductase inhibitor, preferably dutasteride, or pharmaceutically acceptable salts thereof, as active ingredient, for use in a method for treating or preventing androgenetic alopecia, wherein the method comprises a daily administration for at least more than one day of a total daily amount of the active ingredient from 0.015 µg to 0.60 µg, throughout the topical administration of a pharmaceutical composition.

### BRIEF DESCRIPTION OF THE FIGURES

FIG. 1 Contact angle measurement for different topical pharmaceutical formulations according to the first aspect invention.
FIG. 2 In-vitro release test results for the topical formulations GLN-8, GLN-17, GLN-19 and GLN-21 , according to the first aspect of the invention.
FIG. 3 In-vitro release test results for the topical formulations GLN-7, GLN-8 and GLN-18, according to the first aspect of the invention.
FIG. 4 Confocal image of a human skin explant in the ex-vivo penetration assay regarding topical pharmaceutical formulation GLN-8.
FIG. 5 Confocal image of a human skin explant in the ex-vivo penetration assay regarding topical pharmaceutical formulation GLN-17.
FIG. 6 Confocal image of a human skin explant in the ex-vivo penetration assay regarding topical pharmaceutical formulation GLN-21.
FIG. 7 Confocal image of a human skin explant in the ex-vivo penetration assay regarding topical pharmaceutical formulation GLN-19.
FIG. 8 Quantification by HPLC of dutasteride in the different strip test groups regarding the ex-vivo tape stripping assay for the topical formulations GLN-7, GLN-8 and GLN-18, according to the first aspect of the invention.
FIG. 9 Cumulative dutasteride in the epidermis and dermis in the ex-vivo tape stripping assay for the topical formulations GLN-7, GLN-8 and GLN-18, according to the first aspect of the invention.

### DETAILED DESCRIPTION OF THE INVENTION

As used in the present disclosure, the following words, phrases, and symbols are generally intended to have the meanings as set forth below, except to the extent that the context in which they are used indicates otherwise.

All percentages are expressed by weight (w/w) and as used herein are referred to the total weight of the composition, unless specifically noted otherwise.

The term "active ingredient" as used herein refers to a therapeutically active compound, as well as any pharmaceutically acceptable hydrates and solvates of the compound.

The term "pharmaceutically acceptable" as used herein indicates that the substance or composition must be compatible chemically and/or toxicologically, with the other ingredients in the composition, and/or the mammal being treated therewith.

The term "topical" as used herein is intended to mean that the route of administration of the pharmaceutical composition is via application onto the skin.

The term "stable" as used herein refers to any pharmaceutical composition comprising the active ingredient having a sufficient physical and chemical stability to allow storage under any of the general storage conditions as defined by ICH Q1A (R2).

The term "nanoparticles" as used herein refers to particles ranging between 1-999 nm in diameter.

The term "hydrodynamic diameter" (Dₕ) as used herein refers to the diameter of a hypothetical solid sphere that diffuses with the same speed as the particle being measured in the same solution. Thus, the hydrodynamic diameter as used herein is an estimation of the size or size distribution of the particles being measured. The hydrodynamic diameter of the particles was determined by intensity by Dynamic Light Scattering (DLS) using a Beckman Coulter^{®} N4 Plus (Beckman Coulter s.r.l. Milan, Italy). The hydrodynamic diameter for each solution was determined on 6 runs of three different samples at an angle of 90° and run time of 200 s at 20 °C. The term "polydispersity index" (P.I.) as used herein refers to a measure of the heterogeneity of the topical pharmaceutical composition based on particle size, determined by the abovementioned DLS equipment.

The term "entrapment efficiency" as used herein refers to the percentage of active ingredient loaded in the nanoparticles with respect to the one added during the preparation of the formulation. The amount of active ingredient was determined by HPLC analysis. In order to quantify the amount of active ingredient loaded in the nanoparticles, aliquots of the pharmaceutical compositions were filtered to remove unloaded drug, and the nanoparticles separated. Then, to ensure complete destruction of the nanoparticles and release of the incorporated drug, the filtrate was diluted with methanol before the HPLC analysis.

The entrapment efficiency of dutasteride was calculated using the following equation: Entrapment efficiency, EE% _ (mass of drug in micelles)/(mass of drug added in the formulation) × 100

The term "drug loading" as used herein refers to the concentration of the active ingredient in the nanoparticles. The drug loading was determined by HPLC analysis.

The quantitative determination of dutasteride by HPLC analysis was carried out using an HPLC-UV apparatus consisting of an LC-20AD Shimadzu system (Shimadzu Corp., Kyoto. Japan) with SPD-10A UV detector equipped with SIL-10AD VP autosampler and a computer integrating system (CR4A). The injection valve was a Rheodyne with a capacity of 20 µl A reverse-phase C18 column (Kinetex 5u. C18, 100 Å, 150x4.6mm, Phenomenex, Torrance, CA, USA) connected to a KJ0-4282 safeguard (Phenomenex^{®}. Italy) was used for the analysis. The isocratic elution was performed using a mobile phase consisting of a mixture of CH3CN/H2O (60/40). The detection wavelength was 235 nm, the flow was 1 ml/min and the retention time was 10 minutes. The amount of dutasteride in the samples was determined by comparison with appropriate external standard curves obtained applying the least square linear regression analysis.

The term "skin penetration enhancer" as used herein refers to chemical compounds that can facilitate the penetration of active pharmaceutical ingredients into or through the skin.

The term "sebum-interacting" as used herein refers to agents that interact with sebum to decrease the barrier resistance of the skin.

The term "sebum-regulating" as used herein refers to agents that regulate the production of sebum in the skin.

The term "rheological agent" as used herein refers to agents that can modify the rheological properties of the pharmaceutical composition.

The term "viscosifier" as used herein refers to agents that can increase the viscosity of the pharmaceutical composition.

The term "release profile" as used herein refers to the amount of active ingredient which is released from the nanoparticles over time.

The term "tocopherol derivative surfactant" as used herein refers to derivatives of tocopherol which can be used as surfactants.

The term "oil" as used herein refers to any nonpolar chemical substance that is composed primarily of hydrocarbons, it is liquid at room temperature, and is hydrophobic and lipophilic. Non-limiting examples include castor oil, liquid paraffin, medium chain triglycerides and white oil.

The terms "alcohol-free" or "free of alcohol" as used herein are intended to mean that the amount or type of alcohol present, if any, in the pharmaceutical composition is insufficient to cause drying, irritation or any adverse effect on the user's skin. Examples of alcohols are ethanol, methanol, ethyl alcohol, isopropyl alcohol and/or benzyl alcohol.

The term "monophasic" as used herein refers to a liquid pharmaceutical composition containing two or more components in one phase system.

The term "phospholipid-based surfactant" as used herein refers to compounds or mixtures of compounds selected from a class of surfactants that are derived from phospholipids.

The term "hydrophilic-lipophilic balance" (HLB) of a surfactant as used herein refers to a measure of its degree of hydrophilicity or lipophilicity. The term "hydrophilic" as used herein refers to a surfactant having an HLB value of more than 10.

The term "pH adjuster" as used herein refers to pharmaceutically acceptable excipients which are added to the solution of the active agent to adjust the pH to a certain value. Such pH adjusters can be alkaline or acid agents and may comprise inorganic salts as well as organic acids or salts of organic acids. Additionally, the pH adjusters may be present in the form of a buffer.

The term "stabilizer" as used herein refers to agents that help to maintain the initial physical and chemical properties of the pharmaceutical composition.

The term "preservative" as used herein refers to agents that delay the growth of microorganisms or prevent deterioration of quality during manufacture and distribution.

The "nanoparticles comprise the active ingredient" means that said nanoparticles may totally or at least partially comprise the active ingredient of the pharmaceutical composition.

All percentages, parts and ratios herein used are by weight unless specifically noted otherwise. As used herein, the term "about" refers preferably to a range that is ±10%, preferably ±5%, or more preferably ±1% of a value with which the term is associated.

Unless otherwise indicated, all the analysis methods are carried out according to the European Pharmacopoeia 10th edition.

The inventors have found out that, the nanoparticles of the topical pharmaceutical compositions for the treatment and/or prevention of androgenetic alopecia according to the first aspect of the invention accumulate in the hair follicles when applied onto the skin and show excellent release profile. Surprisingly, the active agent is solubilized in the alcohol-free topical pharmaceutical composition while systemic absorption, and thus the adverse effects resulting from it, is reduced.

According to the first aspect, the invention refers to a topical pharmaceutical composition for the treatment and/or prevention of androgenetic alopecia comprising:
a) 0.03-0.3% w/w of an active ingredient, preferably 0.06-0.1% w/w, more preferably 0.09 w/w, wherein the active ingredient is a 5-alpha reductase inhibitor, pharmaceutically acceptable salts or mixtures thereof; and
b) 1-15% w/w of a first surfactant, preferably 2-10% w/w, more preferably 3-5% w/w;
wherein the first surfactant is forming nanoparticles having a hydrodynamic diameter between 3-700 nm measured by DLS, wherein said nanoparticles comprise the active ingredient; and wherein the composition is free of alcohol.

In an embodiment according to the first aspect, the first surfactant is a tocopherol derivative surfactant or a phospholipid-based surfactant.

In another embodiment, the active ingredient is selected from dutasteride, finasteride, pharmaceutically acceptable salts or mixtures thereof.

In another embodiment, the topical pharmaceutical composition further comprises at least 60% w/w of water, preferably at least 70% w/w of water. In a preferred embodiment, the topical pharmaceutical composition is an aqueous topical pharmaceutical composition. In another embodiment, the topical pharmaceutical composition is monophasic.

In another embodiment, the composition is oil-free. The composition being oil-free avoids unpleasant user experience after application e.g., oily or greasy scalp, which would otherwise reduce patient adherence to the treatment.

In another embodiment, the first surfactant is a tocopherol derivative surfactant. Examples of suitable tocopheryl derivative surfactants are tocopheryl acetate, tocopheryl phosphate and tocopheryl polyethylene glycol succinate (TPGS). In a preferred embodiment, the first surfactant is TPGS.

In another embodiment, the nanoparticles have a hydrodynamic diameter between 3-100 nm measured by DLS, preferably 5-60 nm measured by DLS, more preferably 5-30 measured by DLS.

In another embodiment, the first surfactant is TPGS, and the nanoparticles have a hydrodynamic diameter between 3-100 nm measured by DLS, preferably 5-60 nm measured by DLS, more preferably 5-30 measured by DLS.

In another embodiment, the first surfactant is a phospholipid-based surfactant. Examples of suitable phospholipid based surfactants are phosphatidylcholine, phosphatidylglycerol, phosphatidylethanolamine, phosphatidylinositol, phosphatidylserine, phosphatidyl ceteareth alcohols and lecithin. In another preferred embodiment, the first surfactant is phosphatidylcholine.

In another embodiment, the nanoparticles have a hydrodynamic diameter between 350-700 nm measured by DLS, preferably 350-650 nm measured by DLS, more preferably 400-600 nm measured by DLS.

In another embodiment, the first surfactant is phosphatidylcholine, and the nanoparticles have a hydrodynamic diameter between 350-700 nm measured by DLS, preferably 350-650 nm measured by DLS, more preferably 400-600 nm measured by DLS.

In another embodiment, the topical pharmaceutical composition comprises at least 70% w/w of glycerin.

In another embodiment, the topical pharmaceutical composition further comprises a second surfactant, preferably the second surfactant is a hydrophilic non-ionic surfactant. Suitable hydrophilic non-ionic surfactants are selected from the group consisting of polyglycerized fatty acids, alcohol-oil transesterification products, propylene glycol fatty acid esters, mono and diglycerides, sterol derivatives, PEG-sorbitan fatty acid esters, polyethylene glycol alkyl ethers, sugar esters, PEG-alkylphenol, polyoxyethylene-polyoxypropylene block copolymers, sorbitan fatty acid esters, lower alcohol fatty acid esters, or mixtures thereof. Preferably, the second surfactant is polyoxyl 35 castor oil, t-octylphenoxypolyethoxyethanol, or mixtures thereof.

In another embodiment, the pharmaceutical composition comprises 0.02-20% w/w of the second surfactant, preferably 5-15% w/w, more preferably 7-15% w/w.

In another embodiment, the pharmaceutical composition further comprises a skin penetration enhancer. Any suitable penetration enhancer may be used. Suitable examples include p azelaic acid, caprylic/capric triglycerides, cetyl alcohol, decyl oleate, diethylhexyl carbonate, glycerin, isopropyl palmitate, lecithin, polysorbate 20, propylene glycol dicaprylate/dicaprate, diethylene glycol monoethyl ether or mixtures thereof, more preferably diethylene glycol monoethyl ether.

In another embodiment, the pharmaceutical composition comprises 3-15% w/w of the skin penetration enhancer, more preferably 5-10% w/w.

In another embodiment, the pharmaceutical composition further comprises a sebum-interacting substance and/or a sebum-regulating substance. Any suitable sebum-interacting substance and/or a sebum-regulating substance may be used. Suitable examples of sebu-interacting substances are alpha-hydroxy acids, beta-hydroxy acids, azelaic acid, niacinamide, zinc, glycine or mixtures thereof, more preferably glycine.

In another embodiment, the pharmaceutical composition comprises 0.1-5% w/w, more preferably, 0.1-1% w/w, of the sebum-interacting substance and/or a sebum-regulating substance.

In another embodiment, the pharmaceutical composition further comprises a rheological agent. Any suitable rheological agents may be used. Suitable rheological agents are viscosifiers. Preferred viscosifiers are selected from carbomers, cellulose derivatives, xanthan gum, guar gum, polyethylene glycols, glycosaminoglycans or mixtures thereof.

In another embodiment, the pharmaceutical composition comprises 0.1-15% w/w of the rheological agent, preferably 0.5-10% w/w, more preferably 1-5% w/w.

In another embodiment, the composition may optionally comprise a preservative agent, pH adjuster and/or stabilizer. Examples of suitable pH adjusters are Citric Acid / Sodium Citrate, Lactic Acid / Sodium Lactate, Phosphoric Acid / Sodium Phosphate, Sodium Hydroxide, Hydrochloric Acid, Acetic Acid / Sodium Acetate. Examples of suitable stabilizers may be preservatives such as parabens, benzalkonium chloride, sorbic acid, sodium benzoate, benzoic acid, benzyl alcohol and potassium sorbate; antioxidants such as tocopherol, butylated hydroxytoluene (BHT) and/or ascorbic acid; chelating agents such as EDTA; and/or silicones such as dimethicone and/or cyclopentasiloxane.

In another embodiment, the pharmaceutical composition comprises between 0.01-2% w/w of the preservative agent, preferably 0.01-1% w/w.

According to the second aspect, the method for the preparation of the topical pharmaceutical composition according to the first aspect of the invention comprises the steps of:
a) heating the first surfactant between 40-60°C;
b) adding the active ingredient, wherein the active ingredient is a 5-alpha reductase inhibitor, pharmaceutically acceptable salts or mixtures thereof;
c) adding water between 40-60ºC; and
d) stirring at room temperature.

In an embodiment of the second aspect, the active ingredient is selected from dutasteride, finasteride, pharmaceutically acceptable salts or mixtures thereof.

In another embodiment, the first surfactant is a tocopheryl derivative surfactant, preferably TPGS.

In another embodiment, a second surfactant and/or a skin penetration enhancer is added in step a) or before step b).

In another embodiment, the sebum-interacting substance and/or a sebum-regulating substance is added in step a) and/or c).

In another embodiment, the temperature in step a) and c) is about the same temperature.

In another embodiment, step h) comprises stirring at least 1h, preferably between 3-48h, more preferably between 12-48h.

According to the third aspect, the method for the preparation of the topical pharmaceutical composition according to the first aspect of the invention comprises the steps of:
a) dissolving the first surfactant;
b) adding the active ingredient, and
c) continue stirring.

In an embodiment of the third aspect, the active ingredient is selected from dutasteride, finasteride, pharmaceutically acceptable salts or mixtures thereof.

In another embodiment, the first surfactant is a phospholipid-based surfactant, preferably phosphatidylcholine.

In another embodiment, a second surfactant and/or a skin penetration enhancer is added in step a) or b).

In another embodiment, the first surfactant is dissolved in the skin penetration enhancer, preferably in diethylene glycol monoethyl ether.

In another embodiment, glycerine is added after step b).

In another embodiment, the first surfactant is dissolved in water.

In another embodiment, the active ingredient is dissolved in the skin penetration enhancer, preferably in diethylene glycol monoethyl ether, before step b).

In another embodiment, the stirring of step c) is carried out for at least 1h.

According to the fourth aspect, the topical pharmaceutical composition according to the first aspect of the invention is used for the treatment of androgenetic alopecia.

According to the fifth aspect, a 5-alpha reductase inhibitor, preferably dutasteride, or pharmaceutically acceptable salts thereof, as active ingredient, for use in a method for treating or preventing androgenetic alopecia, wherein the method comprises a daily administration for at least more than one day of a total daily amount of the active ingredient from 0.015 µg to 0.60 µg, throughout the topical administration of a pharmaceutical composition.

Further aspects and embodiments of the present invention can be found in the following numbered clauses:
Clause 1. A topical pharmaceutical composition for the treatment and/or prevention of androgenetic alopecia comprising:
   0.03-0.3% w/w of an active ingredient, wherein the active ingredient is a 5-alpha reductase inhibitor, preferably selected from dutasteride, finasteride, pharmaceutically acceptable salts or mixtures thereof; and
   1-15% w/w of a first surfactant, preferably wherein the first surfactant is a tocopherol derivative surfactant or a phospholipid-based surfactant;
   wherein the first surfactant is forming nanoparticles having a hydrodynamic diameter between 3-700 nm measured by DLS, wherein said nanoparticles comprise the active ingredient; and wherein the composition is free of alcohol.
Clause 2. The topical pharmaceutical composition according to clause 1, comprising 0.06-0.1% w/w, preferably 0.09 w/w, of the active ingredient.
Clause 3. The topical pharmaceutical composition according to any of the preceding clauses, comprising 2-10% w/w, preferably 3-5% w/w, of the first surfactant.
Clause 4. The topical pharmaceutical composition according to any of the preceding clauses, wherein the pharmaceutical composition is monophasic.
Clause 5. The topical pharmaceutical composition according to any of the preceding clauses, wherein the first surfactant is a tocopherol derivative surfactant, preferably tocopheryl polyethylene glycol succinate.
Clause 6. The topical pharmaceutical composition according to any of the preceding clauses, wherein the nanoparticles have a hydrodynamic diameter between 3-100 nm measured by DLS, preferably 5-60 nm measured by DLS, more preferably 5-30 measured by DLS.
Clause 7. The topical pharmaceutical composition according to any of the preceding clauses, further comprising at least 60% w/w of water, preferably at least 70% w/w of water.
Clause 8. The topical pharmaceutical composition according to any of the preceding clauses, further comprising a second surfactant, preferably wherein the second surfactant is a hydrophilic non-ionic surfactant.
Clause 9. The topical pharmaceutical composition according to clause 8, wherein the second surfactant is selected from the group consisting of polyglycerized fatty acids, alcohol-oil transesterification products, propylene glycol fatty acid esters, mono and diglycerides, sterol derivatives, PEG-sorbitan fatty acid esters, polyethylene glycol alkyl ethers, sugar esters, PEG-alkylphenol, polyoxyethylene-polyoxypropylene block copolymers, sorbitan fatty acid esters, lower alcohol fatty acid esters, or mixtures thereof.
Clause 10. The topical pharmaceutical composition according to clauses 8 or 9, comprising 0.02-20% w/w of the second surfactant, preferably 5-15% w/w, more preferably 7-15% w/w.
Clause 11. The topical pharmaceutical composition according to any of the preceding clauses, further comprising a skin penetration enhancer, preferably selected from azelaic acid, caprylic/capric triglycerides, cetyl alcohol, decyl oleate, diethylhexyl carbonate, glycerin, isopropyl palmitate, lecithin, polysorbate 20, propylene glycol dicaprylate/dicaprate, diethylene glycol monoethyl ether or mixtures thereof, more preferably diethylene glycol monoethyl ether.
Clause 12. The topical pharmaceutical composition according to clause 11, comprising 3-15% w/w of the skin penetration enhancer, more preferably 5-10% w/w.
Clause 13. The topical pharmaceutical composition according to any of the preceding clauses, further comprising a sebum-interacting substance and/or a sebum-regulating substance, preferably selected from alpha-hydroxy acids, beta-hydroxy acids, azelaic acid, niacinamide, zinc, glycine or mixtures thereof, more preferably glycine.
Clause 14. The topical pharmaceutical composition according to any of the preceding clauses, wherein the topical pharmaceutical composition is oil-free.
Clause 15. The topical pharmaceutical composition according to any of the preceding clauses, further comprising a preservative agent, pH adjuster and/or stabilizer.
Clause 16. The topical pharmaceutical composition according to any of clauses 1-4, wherein the first surfactant is a phospholipid based surfactant, preferably a phosphatidylcholine derivative.
Clause 17. The topical pharmaceutical composition according to any of clauses 1-4 or 16, wherein the nanoparticles have a hydrodynamic diameter between 350-700 nm measured by DLS, preferably 350-650 nm measured by DLS, more preferably 400-600 nm measured by DLS.
Clause 18. The topical pharmaceutical composition according to any of clauses 1-4 or 16-17, further comprising at least 70% w/w of glycerin.
Clause 19. The topical pharmaceutical composition according to any of clauses 1-4 or 16-18, further comprising a second surfactant, preferably wherein the second surfactant is non-ionic, more preferably wherein the second surfactant is a hydrophilic non-ionic surfactant.
Clause 20. The topical pharmaceutical composition according to clause 19, wherein the second surfactant is selected from the group consisting of polyglycerized fatty acids, alcohol-oil transesterification products, propylene glycol fatty acid esters, mono and diglycerides, sterol derivatives, PEG-sorbitan fatty acid esters, polyethylene glycol alkyl ethers, sugar esters, PEG-alkylphenol, polyoxyethylene-polyoxypropylene block copolymers, sorbitan fatty acid esters, lower alcohol fatty acid esters, or mixtures thereof.
Clause 21. The topical pharmaceutical composition according to clauses 19 or 20, comprising 0.02-20% w/w of the second surfactant, preferably 5-15% w/w, more preferably 7-15% w/w.
Clause 22. The topical pharmaceutical composition according to any of clauses 1-4 or 16-21, further comprising a skin penetration enhancer, preferably selected from azelaic acid, caprylic/capric triglycerides, cetyl alcohol, decyl oleate, diethylhexyl carbonate, glycerin, isopropyl palmitate, lecithin, polysorbate 20, propylene glycol dicaprylate/dicaprate, diethylene glycol monoethyl ether or mixtures thereof, more preferably diethylene glycol monoethyl ether.
Clause 23. The topical pharmaceutical composition according to clause 22, comprising 3-15% w/wof the skin penetration enhancer, more preferably 5-10% w/w.
Clause 24. The topical pharmaceutical composition according to any of clauses 1-4 or 16-23, further comprising a sebum-interacting substance and/or a sebum-regulating substance, preferably selected from alpha-hydroxy acids, beta-hydroxy acids, azelaic acid, niacinamide, zinc, glycine or mixtures thereof, more preferably glycine.
Clause 25. The topical pharmaceutical composition according to any of clauses 1-4 or 16-24, wherein the topical pharmaceutical composition is oil-free.
Clause 26. The topical pharmaceutical composition according to any of clauses 1-4 or 16-25, further comprising a preservative agent, pH adjuster and/or stabilizer.
Clause 27. A method for the preparation of the topical pharmaceutical composition according to any of clauses 1-15 comprising the steps of:
   a) heating the first surfactant between 40-80°C;
   b) adding active ingredient selected from dutasteride, finasteride, pharmaceutically acceptable salts or mixtures thereof;
   c) adding water between 40-80ºC; and
   d) stirring at room temperature.
Clause 28. The method for the preparation of the topical pharmaceutical composition according to clause 27, wherein the first surfactant is TPGS.
Clause 29. The method for the preparation of the topical pharmaceutical composition according to clauses 27 or 28, further comprising adding a second surfactant and/or a skin penetration enhancer in step a) and/or before step b).
Clause 30. The method for the preparation of the topical pharmaceutical composition according to clause 29, wherein the second surfactant is selected from the group consisting of polyglycerized fatty acids, alcohol-oil transesterification products, propylene glycol fatty acid esters, mono and diglycerides, sterol derivatives, PEG-sorbitan fatty acid esters, polyethylene glycol alkyl ethers, sugar esters, PEG-alkylphenol, polyoxyethylene-polyoxypropylene block copolymers, sorbitan fatty acid esters, lower alcohol fatty acid esters, or mixtures thereof; and/or the skin penetration enhancer is selected from azelaic acid, caprylic/capric triglycerides, cetyl alcohol, decyl oleate, diethylhexyl carbonate, glycerin, isopropyl palmitate, lecithin, polysorbate 20, propylene glycol dicaprylate/dicaprate, diethylene glycol monoethyl ether or mixtures thereof.
Clause 31. The method for the preparation of the topical pharmaceutical composition according to any of clauses 27-30, further comprising adding a sebum-interacting substance and/or a sebum-regulating substance, preferably a sebum-interacting substance and/or a sebum-regulating substance selected from alpha-hydroxy acids, beta-hydroxy acids, azelaic acid, niacinamide, zinc, glycine or mixtures thereof, more preferably glycine.
Clause 32. The method for the preparation of the topical pharmaceutical composition according to any of clauses 27-31, wherein the temperature in step a) and c) is about the same temperature.
Clause 33. A method for the preparation of the topical pharmaceutical composition according to any of clauses 1-4 or 16-26 comprising the steps of:
   a) dissolving the first surfactant;
   b) adding the active ingredient selected from dutasteride, finasteride, pharmaceutically acceptable salts or mixtures thereof under stirring; and
   c) continue stirring.
Clause 34. The method for the preparation of the topical pharmaceutical composition according to clause 33, wherein the first surfactant is phospholipid-based surfactant, preferably phosphatidylcholine.
Clause 35. The method for the preparation of the topical pharmaceutical composition according to clause 33 or 34, further comprising adding a second surfactant and/or a skin penetration enhancer is added in step a) and/or b).
Clause 36. The method for the preparation of the topical pharmaceutical composition according to clause 35, wherein the second surfactant is selected from the group consisting of polyglycerized fatty acids, alcohol-oil transesterification products, propylene glycol fatty acid esters, mono and diglycerides, sterol derivatives, PEG-sorbitan fatty acid esters, polyethylene glycol alkyl ethers, sugar esters, PEG-alkylphenol, polyoxyethylene-polyoxypropylene block copolymers, sorbitan fatty acid esters, lower alcohol fatty acid esters, or mixtures thereof; and/or the skin penetration enhancer is selected from azelaic acid, caprylic/capric triglycerides, cetyl alcohol, decyl oleate, diethylhexyl carbonate, glycerin, isopropyl palmitate, lecithin, polysorbate 20, propylene glycol dicaprylate/dicaprate, diethylene glycol monoethyl ether or mixtures thereof.
Clause 37. The method for the preparation of the topical pharmaceutical composition according to clause 36, wherein the first surfactant is dissolved in the skin penetration enhancer, preferably in diethylene glycol monoethyl ether.
Clause 38. The method for the preparation of the topical pharmaceutical composition according to any of clauses 33-37, wherein glycerine is added after step b).
Clause 39. The method for the preparation of the topical pharmaceutical composition according to any of clauses 33-38, wherein the first surfactant is dissolved in water.
Clause 40. The method for the preparation of the topical pharmaceutical composition according to any of clauses 33-39, the active ingredient is dissolved in the skin penetration enhancer, preferably in diethylene glycol monoethyl ether, before step b).
Clause 41. The topical pharmaceutical composition according to any of clauses 1-26 for use in the treatment of androgenetic alopecia.
Clause 42. A 5-alpha reductase inhibitor, preferably dutasteride, or pharmaceutically acceptable salts thereof, as active ingredient, for use in a method for treating or preventing androgenetic alopecia, wherein the method comprises a daily administration for at least more than one day of a total daily amount of the active ingredient from 0.015 µg to 0.60 µg, throughout topical administration of a pharmaceutical composition.

### EXAMPLES

### Example 1. Preparation of the topical pharmaceutical compositions comprising TPGS.

The topical pharmaceutical compositions comprising TPGS as the first surfactant (GLN-1 to GLN-17) were prepared as follows:
a) weighing the necessary amount of TPGS;
b) heating TPGS between about 40-60ºC;
c) adding the necessary amount of dutasteride while stirring to form a mixture;
d) adding water at about the same temperature of the mixture under continuous stirring; and
e) stirring at room temperature.

The necessary amount of additional components such as a second surfactant, skin penetration enhancer and/or sebum-regulating agent (diethylene glycol monoethyl ether (DGME), polyoxyl 35 castor oil (P35), t-octylphenoxypolyethoxyethanol (TRX), Polysorbate 80 (T80), and glycine), if any, was incorporated in the preparation of the topical pharmaceutical formulation as follows:
- DGME, P35, TRX and/or T80 were added during step b); and/or
- glycine was solubilised in water and added in step d).

| **Formulati on** | **Dutasteri de** | **TPGS** | **DGME** | **P35** | **Glycine** | **TRX** | **T80** | **Water** |
|---|---|---|---|---|---|---|---|---|
| GLN-1 | 0,30 | 3,00 | - | - | - | - | - | to 100,00 |
| GLN-2 | 0,30 | 5,00 | - | - | - | - | - | to 100,00 |
| GLN-3 | 0,30 | 5,00 | 5,00 | - | - | - | - | to 100,00 |
| GLN-4 | 0,30 | 5,00 | 10,00 | - | - | - | - | to 100,00 |
| GLN-5 | 0,30 | 5,00 | - | - | 0,10 | - | - | to 100,00 |
| GLN-6 | 0,30 | 5,00 | - | - | 0,50 | - | - | to 100,00 |
| GLN-7 | 0,30 | 5,00 | - | - | 1,00 | - | - | to 100,00 |
| GLN-8 | 0,30 | 3,00 | - | 0,02 | - | - | - | to 100,00 |
| GLN-9 | 0,30 | 3,00 | - | 2,00 | - | - | - | to 100,00 |
| GLN-10 | 0,30 | 1,00 | - | 2,00 | - | - | - | to 100,00 |
| GLN-11 | 0,30 | - | - | 2,00 | - | - | - | to 100,00 |
| GLN-12 | 0,30 | 5,00 | - | - | - | 12,50 | - | to 100,00 |
| GLN-13 | 0,30 | 5,00 | - | - | - | - | 4,00 | to 100,00 |
| GLN-14 | 0,03 | 5,00 | - | - | 1,00 | - | - | to 100,00 |
| GLN-15 | 0,025 | 5,00 | - | - | 1,00 | - | - | to 100,00 |
| GLN-16 | 0,03 | 5,00 | - | 0,02 | - | - | - | to 100,00 |
| GLN-17 | 0,09 | 5,00 | - | 0,02 | - | 12,50 | - | to 100,00 |

The amount of the different components are expressed as percentages by weight (w/w) of the total composition.

### Example 2. Preparation of the topical pharmaceutical compositions comprising a phospholipid-based surfactant.

The topical pharmaceutical compositions (GLN-18 to GLN-20) comprising a phosphatidylcholine as the first surfactant were prepared as follows:
a) weighing the necessary amount of phosphatidylcholine;
b) dissolving phosphatidylcholine in diethylene glycol monoethyl ether and stirring for 1h;
c) adding dutasteride and stirring for 30 min; and
d) slowly adding glycerin and stirring.

The topical pharmaceutical composition (GLN-21) comprising phosphatidylcholine as the first surfactant was prepared as follows:
a) weighing the necessary amount of phosphatidylcholine;
b) dissolving phosphatidylcholine in water;
c) dissolving dutasteride in diethylene glycol monoethyl ether;
d) slowly adding solution obtained in step c) to solution obtained in step b); and
e) stirring.

The following table depicts the quantitative compositions GLN-18 to GLN-21 :

| **Formulation** | **Dutasteride** | **Phosphatidylc holine** | **Glycerin** | **Water** | **DGME** |
|---|---|---|---|---|---|
| GLN-18 | 0,03 | 5,00 | 70,00 | - | to 100,00 |
| GLN-19 | 0,09 | 5,00 | 70,00 | - | to 100,00 |
| GLN-20 | 0,15 | 5,00 | 70,00 | - | to 100,00 |
| GLN-21 | 0,09 | 5,00 | - | 70,00 | to 100,00 |

The amount of the different components are expressed as percentages by weight (w/w) of the total composition.

### Example 3. Physicochemical characterization of the pharmaceutical formulations

The nanoparticles in the topical pharmaceutical compositions were characterised with respect to size distribution (hydrodynamic diameter), polydispersity index (P.I.) and drug loading. The hydrodynamic diameter and P.I. were determined by DLS and the drug loading by HPLC.

| **Formulations** | **Dh (nm)** | **P.I.** | **Drug loading mg/ml** |
|---|---|---|---|
| GLN-1 | 19,90 | 0,93 | 0,223 |
| GLN-2 | 13,80 | 0,68 | 0,343 |
| GLN-3 | 19,60 | 0,78 | 0,457 |
| GLN-4 | 29,20 | 1,01 | 0,127 |
| GLN-5 | 12,10 | 0,35 | 0,272 |
| GLN-6 | 19,50 | 0,86 | 0,222 |
| GLN-7 | 13,00 | 0,62 | 0,266 |
| GLN-8 | 11,90 | 0,23 | 0,090 |
| GLN-9 | 12,90 | 0,49 | 0,191 |
| GLN-10 | 13,70 | 0,24 | 0,060 |
| GLN-12 | 5,80 | 0,79 | 0,402 |
| GLN-13 | 10,90 | 0,69 | 0,198 |
| GLN-15 | 13,14 | 0,32 | 0,240 |
| GLN-16 | 13,48 | 0,45 | 0,292 |
| GLN-17 | 7,34 | 0,65 | 0,354 |
| GLN-18 | 594,8 | 1,18 | 0,317 |
| GLN-19 | 497,5 | 1,16 | 0,868 |
| GLN-20 | 589,5 | 1,30 | 1,825 |
| GLN-21 | 357,6 | 0,62 | 1,818 |

### Example 4. Physicochemical characterization of the pharmaceutical formulations

The ability of some of the prepared topical pharmaceutical formulations to interact with the skin surface and, in particular, its affinity for the sebum-rich hydrolipidic film that can be found on the top of the follicular openings has been determined by measuring the contact angle that is formed by a predetermined amount of formulation dropped onto an artificial Skin Surface Film Liquid (SSFL). The artificial Skin Surface Film Liquid (SSFL) was prepared by mixing in a 1:1 ratio an artificial sebum with an artificial sweat, which composition is as follows:

| | | **Composition** | **%w/w** | | |
|---|---|---|---|---|---|
| **ARTIFICIAL SEBUM** | | | **ARTIFICIAL SWEAT** | | |
| Squalene | | 15.00 | Water | | 93.93 |
| Paraffin wax | | 23.60 | Sodium chloride | | 2.00 |
| Olive oil | 10.00 | | Lactic add | | 1.50 |
| Coconut oil | | 25.00 | Urea | | 0.50 |
| Avocado oil | | 10.00 | Acetic acid | | 0.25 |
| Palmitic acid | | 10.00 | Ammonium chloride | 1.75 | |
| Oleic acid | | 1.40 | Glycin | 0.10 | |
| Cholesterol | | 5.00 | *to pH 4.7 with lactic acid* | | |

An optical contact angle-measuring instrument (OCA 15. DataPhysics Instrument GmbH. Germany) has been used to measure the static contact angles. The system comprises a high resolution CCD video camera and a six-fold power zoom lens with integrated fine focusing; the images are recorded and analysed by SCA 20 software. The sessile drop method was used which has the following steps: dropping a known volume (6 µL) of formulation on the film surface; waiting until the spreading droplet achieves an equilibrium state, taking the image and analysing the data by the Laplace-Young fit method.

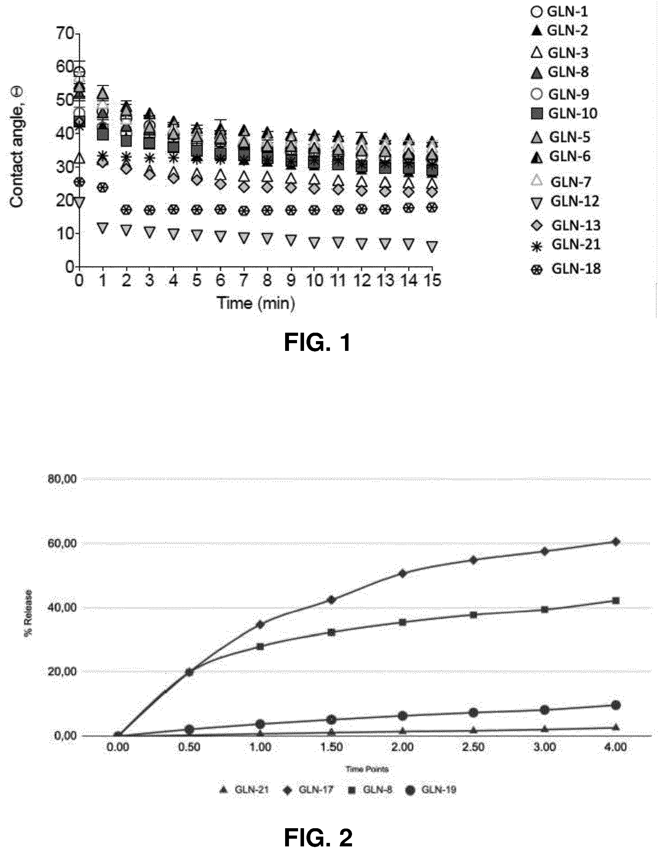

The results of the optical contact angle measurement can be seen in FIG.1. All the formulations tested showed good wettability of the artificial hydrolipidic film, with angles below 60° for the first contact times. The GLN-17 contact angle is not shown, however, it was lower than that of GLN-12.

### Example 5. In-vitro release test

A comparative in-vitro release test for the topical pharmaceutical formulations GLN-8, GLN-17, GLN-19 and GLN-21 was carried out in a Franz diffusion cell assay. These formulations were selected as representatives of the total prepared formulations. The Franz diffusion cell system parameters and sampling time points are provided in below table:

| | |
|---|---|
| **Franz Diffusion Cell System** | Phoenix DB-6 Manual Diffusion cell system |
| **Sampling Time Points** | 0.00, 0.50, 1.00, 1.50, 2.00, 3.00, 4.00 Hrs |
| **Synthetic Membrane (mdi MEMBRANE FILTERS)** | Polyethersulfone membrane Disc Filters; Pore Size: 0.2 µm, Diameter: 25 mm |
| **Donor chamber diameter** | 9 mm |
| **Surface area** | 0.6362 cm² |
| **Receptor medium** | Water :Ethanol (70:30%) |
| **Stirring speed** | 700 rpm |
| **Cell receptor medium temperature** | 32°C ± 1°C |
| **Receptor medium volume** | 10 mL |
| **Sample application volume** | 0.2 mL |
| **Aliquot volume** | 0.25 mL |
| **Aliquot replacement volume** | 0.25 mL |
| **Nº of aliquots to be withdrawn (cells)** | 06 |
| **Environmental condition** | Room temperature - Not More Than 21°C± 2°C And Relative humidity: 50% ± 20% |
| **Quantification** | HPLC Analysis |

The cumulative amount of dutasteride released by each of the GLN-8, GLN-17, GLN-19 and GLN-21 formulations at the different times can be found in FIG. 2. All tested formulations showed release of the active ingredient. Formulations GLN-8 and GLN-17 showed a faster release profile than GLN-19 and GLN-21.

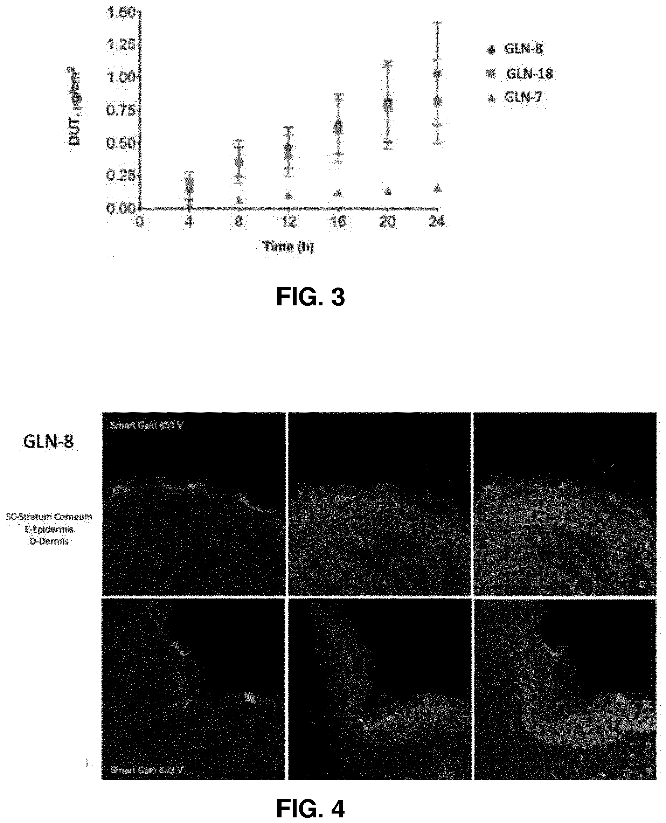

In another set of experiments the amount of dutasteride per area unit was calculated for formulations GLN-8, GLN-18 and GLN-7 during 24h. The results can be found in FIG 3.

### Example 6. Ex-vivo penetration assay

The ability of the GLN-8, GLN-17, GLN-19 and GLN-21 formulations to penetrate the skin and/or hair follicle was analysed by fluorescent penetration assay using confocal microscopy after topical application on human skin explants. The assay comprises the following steps: a dye delivery solution (Vybrant DiO) was added to the liposomes and incubated 30 min. Products were topically applied on human skin for 2 hours. After the incubation time, treated skin fragments were sectioned into pieces of approximately 0.5 cm2, and the adjacent fat portion was removed. Sections were introduced into scaffolds and embedded in optimal cutting temperature (O.C.T.). Immediately after, scaffolds were introduced in a container with isopentane frozen in liquid nitrogen. Frozen samples were stored at -80C. The cryosections were fixed with 4% paraformaldehyde (PFA) 15 minutes at room temperature, washed with phosphate buffered saline (PBS) and incubated with Phalloidin 1:200 for 30 min, followed by the incubation with 1µg/ml 4',6-diamidino-2-fenilindol (DAPI) for 10 minutes. Once stained, tissue samples were mounted using fluorescence preservation medium. Samples were anayzed with Leica confocal microscope at 63x objective and optical zoom. Nuclei were stained with DAPI, and F-actin filaments were stained with Phalloidin-Alexa633 to visualize the structure of the skin. The localization of DiO-labelled products within the skin layers was examined by confocal microscopy. In all images obtained DiO was excited at 488nm; Phalloidin-Alexa633 was excited at 633 nm and DAPI was excited at 405nm.

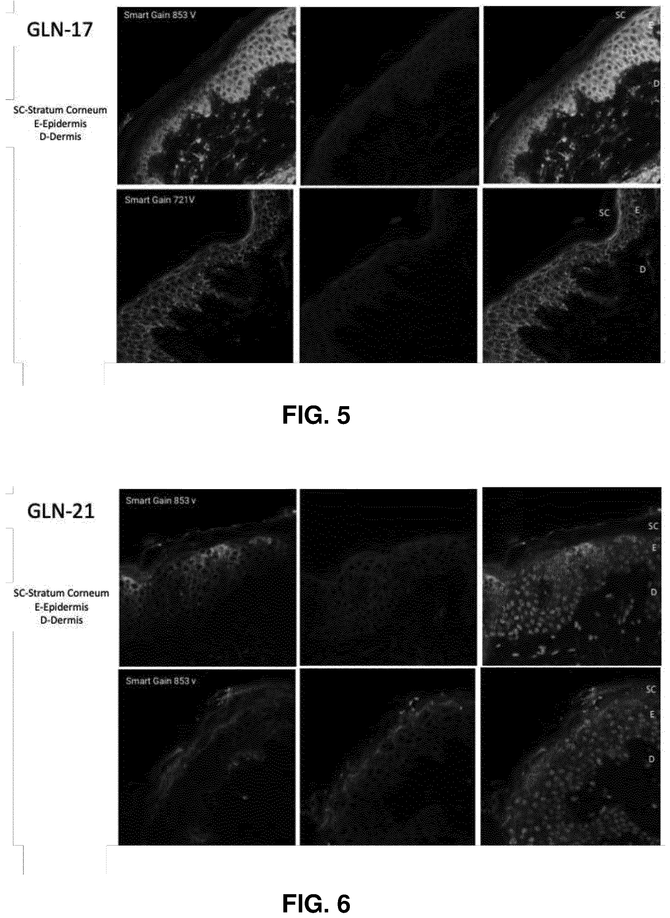

The results of the ex-vivo penetration assay for each of the tested formulations can be found in FIG. 4-7 showing different penetration depths. The formulations GLN-17, GLN-19 and GLN-21 show penetration through three layers of the skin, i.e., the stratum corneum until the epidermis and dermis. The GLN-8 formulation releases the active ingredient at the stratum corneum.

### Example 7. Ex-vivo tape stripping

The ability of the GLN-8, GLN-17, GLN-19 and GLN-21 formulations to penetrate the skin and/or hair follicle was also assessed by ex-vivo tape stripping assay. Briefly, the assay was carried out as follows:
A tape stripping procedure was conducted using 26 sequential adhesive films, specifically D-Squame tapes, for each experimental trial. The ex-vivo tape stripping was executed under standardized conditions to ensure consistency. To account for potential influences on the quantity of removed skin tissue, factors such as the intensity and duration of pressure application, as well as the velocity of tape removal, were carefully considered in order to establish a reproducible working procedure. Porcine ears were extended and affixed to Styrofoam plates using needles. Following the placement of the initial adhesive film on the skin, its outline was marked with a permanent marker to ensure subsequent tape stripping occurred in the exact same area of skin. Pressure was applied using a vinyl-gloved thumb, and a balance was employed during the experiment to maintain a constant pressure of 19 N (2 kg). This relatively high pressure was essential for achieving consistent quantities of removed skin tissue. After applying the specified pressure for a duration of 3 seconds, the tape was swiftly removed in a single motion. Subsequently, the amount of dutasteride adhering to the tape was determined through HPLC analysis. The cumulative quantity of dutasteride was then utilized to assess its penetration into the skin.

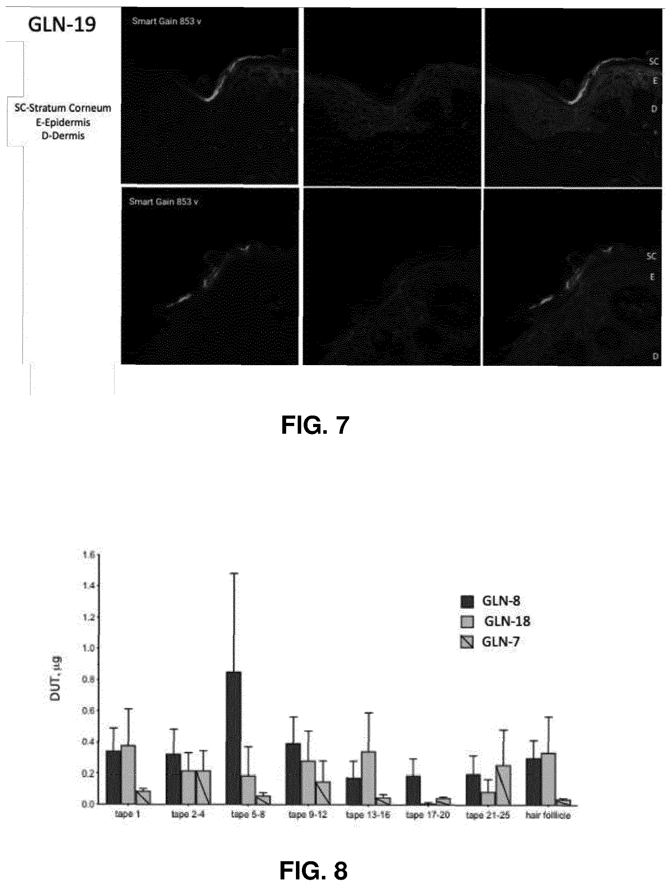

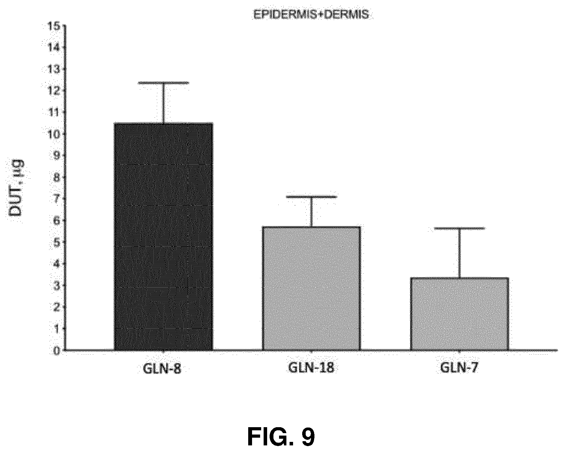

The obtained results can be found in FIG. 8 and 9. As it can be seen, GLN-8 formulation released substantially higher amounts of dutasteride in the epidermis and dermis than GLN-18 and GLN-7.

## Claims

1. A topical pharmaceutical composition for the treatment and/or prevention of androgenetic alopecia comprising:
a) 0.03-0.3% w/w of an active ingredient, preferably 0.06-0.1% w/w, more preferably 0.09 w/w, wherein the active ingredient is a 5-alpha reductase inhibitor, pharmaceutically acceptable salts or mixtures thereof; and
b) 1-15% w/w of a first surfactant, preferably 2-10% w/w, more preferably 3-5% w/w;
wherein the first surfactant is forming nanoparticles having a hydrodynamic diameter between 3-700 nm measured by DLS, wherein said nanoparticles comprise the active ingredient; and wherein the composition is free of alcohol.

2. The topical pharmaceutical composition according to claim 1, wherein the first surfactant is a tocopherol derivative surfactant or a phospholipid-based surfactant.

3. The topical pharmaceutical composition according to any of the preceding claims, wherein the active ingredient is selected from dutasteride, finasteride, pharmaceutically acceptable salts or mixtures thereof.

4. The topical pharmaceutical composition according to any of the preceding claims, further comprising at least 60% w/w of water, preferably at least 70% w/w of water.

5. The topical pharmaceutical composition according to any of the preceding claims, wherein the first surfactant is TPGS, and wherein the nanoparticles have a hydrodynamic diameter between 3-100 nm measured by DLS, preferably 5-60 nm measured by DLS, more preferably 5-30 measured by DLS.

6. The topical pharmaceutical composition according to any of claims 1-3, wherein the first surfactant is phosphatidylcholine, and wherein the nanoparticles have a hydrodynamic diameter between 350-700 nm measured by DLS, preferably 350-650 nm measured by DLS, more preferably 400-600 nm measured by DLS.

7. The topical pharmaceutical composition according to any of claims 1-3 or 6, further comprising at least 70% w/w of glycerin.

8. The topical pharmaceutical composition according to any of the preceding claims, further comprising a second surfactant, preferably wherein the second surfactant is a hydrophilic non-ionic surfactant.

9. The topical pharmaceutical composition according to claim 8, comprising 0.02-20% w/w of the second surfactant, preferably 5-15% w/w, more preferably 7-15% w/w.

10. The topical pharmaceutical composition according to any of the preceding claims, further comprising a skin penetration enhancer, preferably selected from azelaic acid, caprylic/capric triglycerides, cetyl alcohol, decyl oleate, diethylhexyl carbonate, glycerin, isopropyl palmitate, lecithin, polysorbate 20, propylene glycol dicaprylate/dicaprate, diethylene glycol monoethyl ether or mixtures thereof, more preferably diethylene glycol monoethyl ether.

11. The topical pharmaceutical composition according to claim 10, comprising 3-15% w/w of the skin penetration enhancer, more preferably 5-10% w/w.

12. The topical pharmaceutical composition according to any of the preceding claims, further comprising a sebum-interacting substance and/or a sebum-regulating substance, preferably selected from alpha-hydroxy acids, beta-hydroxy acids, azelaic acid, niacinamide, zinc, glycine or mixtures thereof, more preferably glycine.

13. A method for the preparation of the topical pharmaceutical composition according to any of the preceding claims comprising the steps of:
a) heating the first surfactant between 40-60°C;
b) adding the active ingredient, wherein the active ingredient is a 5-alpha reductase inhibitor, pharmaceutically acceptable salts or mixtures thereof;
c) adding water between 40-60ºC; and
d) stirring at room temperature.

14. The topical pharmaceutical composition according to any of claims 1-12 for use in the treatment of androgenetic alopecia.

15. A 5-alpha reductase inhibitor, preferably dutasteride, or pharmaceutically acceptable salts thereof, as active ingredient, for use in a method for treating or preventing androgenetic alopecia, wherein the method comprises a daily administration for at least more than one day of a total daily amount of the active ingredient from 0.015 µg to 0.60 µg, throughout topical administration of a pharmaceutical composition.
